# EUROPEAN PATENT SPECIFICATION

(11) **EP 2 163 253 B1**
(45) Date of publication and mention of the grant of the patent: **17.07.2013**
(21) Application number: 08164365.2
(22) Date of filing: 15.09.2008
(51) Int. Cl.: A61K 36/906, A61P 25/00, A61P 29/00, A61P 37/06

(54) **Extracts from the plant Hornstedtia scyphifera and immunosuppressive effects thereof**
Extrakte von der Pflanze Hornstedtia scyphifera und deren immunsuppressive Wirkungen
Extraits de la plante Hornstedtia scyphifera et ses effets immunosuppresseurs

(43) Date of publication of application: 17.03.2010
(73) Proprietor: ULLRICH, Oliver, 88045 Friedrichshafen (DE)
(72) Inventor: Zipp, Frauke, 13509 Berlin (DE); Ullrich, Oliver, 88045 Friedrichshafen (DE)
(74) Representative: Ziebig, Marlene

(56) References cited:
- SIRAT HASNAH M ET AL: "The distribution of labdane diterpenes in the Zingiberaceae of Malaysia" 1994, PHYTOCHEMISTRY (OXFORD), VOL. 36, NR. 3, PAGE(S) 699-701 , XP009121109 ISSN: 0031-9422 * table 1 *
- DATABASE WPI Week 199723 Thomson Scientific, London, GB; AN 1997-255467 XP002540088 & JP 09 087193 A (TERUMO CORP) 31 March 1997 (1997-03-31)
- SAKAI SHOKO ET AL: "Systematic studies of Bornean Zingiberaceae IV. Alpinioideae of Lambir Hills, Sarawak" EDINBURGH JOURNAL OF BOTANY, UNIVERSITY PRESS, UK, vol. 60, no. 2, 1 July 2003 (2003-07-01), pages 181-216, XP009121107 ISSN: 0960-4286

## Description

### Field of the invention

The present invention concerns extracts from the plant *Hornstedtia scyphifera*, pharmaceutical compositions containing said extracts, methods of manufacturing said extracts and uses of said extracts in prophylaxis and treatment of inflammatory and/or neurological diseases.

### Background

Since centuries plant extracts have been used by virtually all human cultures e.g. for medical and related purposes. Up to now, such plant extracts have been used mainly in traditional medicine, but in recent years their importance as a basis for prophylaxis and treatment of a number of diseases is more and more recognized by modern scientists, hospitals and medical practitioners. A number of plants, whose usefulness was not known until recently and which have been regarded as exotic and marginal, are nowadays used broadly, particularly in the medical field. The search for new active means and substances derivable from natural resources like e.g. plants plays a central role in the development of pharmaceuticals. Without the results from this research, some disease areas would remain completely orphan and without any suitable therapeutic option (Verdine G. Nature (1996) 384, 11-13). One prominent example of an active agent that is used widely in medical treatment today and which has been derived originally from natural resources is the cytostatic compound Taxol. Taxol can be derived from the bark of the yew tree *Taxus brevifolia*.

Apart from recent successes, there remains the need to provide further extracts from plants that have not been described previously for a medical use and to make ingredients of said plants usable as active agents in pharmaceutical compositions.

### Description of the invention

According to the present invention, an extract from the plant *Hornstedtia scyphifera* is provided obtainable by:
- extracting leaf and bark and/or root material of *Hornstedtia scyphifera* with methanol;
- partitioning the methanol extract with 1:1 mixture of chloroform and water;
- removing undissolved material;
- freeing chloroform extract from solvent and partitioning the extract with a 1:1 mixture of hexan and 20% aqueous methanol;
- removing undissolved material;
- separating the remainder in a hexan extract and an aqueous methanol extract;
- drying the methanolic extract and resolving the dried extract in ethanol.

Further embodiments of the invention are defined in the claims.

*Hornstedtia scyphifera*, also called Greater Spindle Ginger, is a perennial herbal plant with creeping rhizomes that are branched and woody, robust pseudostems and leafs, where the leaf blade is lanceolate.

In the sense of the present application, the term "plant" encompasses both the whole plant and parts of a plant like e.g. root, bark, leaf, stipe, fruit, seed, flower and any combinations thereof.

In the sense of the present invention, the term "extract" encompasses any substance or mixture of substances derivable from a plant or parts thereof by one or more steps of an extraction method. Extracts according to the invention can be e.g. in a liquid form, in a solid form, can be a powder or a micronised fraction or can be in any other state.

The extract according to the invention can be manufactured by common methods for preparing plant extracts that are well known to the person skilled in the art. Suitable methods are, but are not limited to, the methods described in Hagers Handbuch der Pharmazeutischen Praxis (5.Auflage, Bd. 2; 1026-1030, Springer Verlag, Berlin-Heidelberg-New York (1991)) and further comprise conventional processes like e.g. maceration, remaceration, digestion, ultrasonic extraction, reverse flow extraction, percolation, repercolation, evacolation, diacolation, and solid-liquid extraction under constant backflow to be performed in a Soxhlet extractor. Fresh plants or parts of plants can be used as raw material as well as dried plants and/or parts of plants. The raw material can be reduced to small pieces before being subjected to the actual extraction. In accordance with the present invention, any means and/or methods known to the skilled person can be applied to reduce fresh or dried plants or parts thereof to smaller pieces, e.g. grinding in a mortar.

Usually the extraction is performed at temperatures between 15 to 100°C, preferably at 20 to 45°C, particularly preferred at approx. body temperature.

In one embodiment of the invention, the extraction is performed under inert gas atmosphere in order to avoid oxidation of ingredients of the raw material or of the extract. Said inert gas atmosphere can be a nitrogen atmosphere.

The extraction time will be determined and adjusted by the skilled person depending on the raw material, the method for extraction, the extraction temperature, the ratio of solvent to raw material and other factors that may have an influence.

If the case may be, the extract obtained after extraction may be subject to further steps like e.g. purification, concentration, dilution and/or decoloration. If desired, undesired ingredients may be separated selectively from the extract. The extracts obtained may be freeze-dried, lyophilised and/or spray-dried.

The extraction can be performed until any desired level of extraction, commonly the extraction will be performed until exhaustion. Typically the extraction of dried plants will yield in the range of 2 to 25, particularly of 10 to 17 weight % dry substance of the extract based on raw material used.

According to the invention, the raw material for the extract comprises aerial parts of *Hornstedtia scyphifera*,namely leafs. In the sense of the present invention, the term "aerial parts" of *Hornstedtia scyphifera* comprises fresh or dried material, that can be harvested from the plant, without to affect significantly its ability to survive and re-grow. The use of aerial parts of the plant offers the advantage that one and the same plant can be harvested multiple times during its life cycle.

In a further aspect of the invention a method of manufacturing of extracts of the invention is provided. This method of manufacturing comprises the step of extracting the plant *Hornstedtia scyphifera* and/or parts thereof as defined in the claims.

Inflammatory and neurologic diseases are often associated or triggered by the presence or increased presence of proinflammatory and/or proliferation inducing cytokines, an increase in the proliferation rate of cells of the immune system, and/or an increase in the release or presence of free radicals.

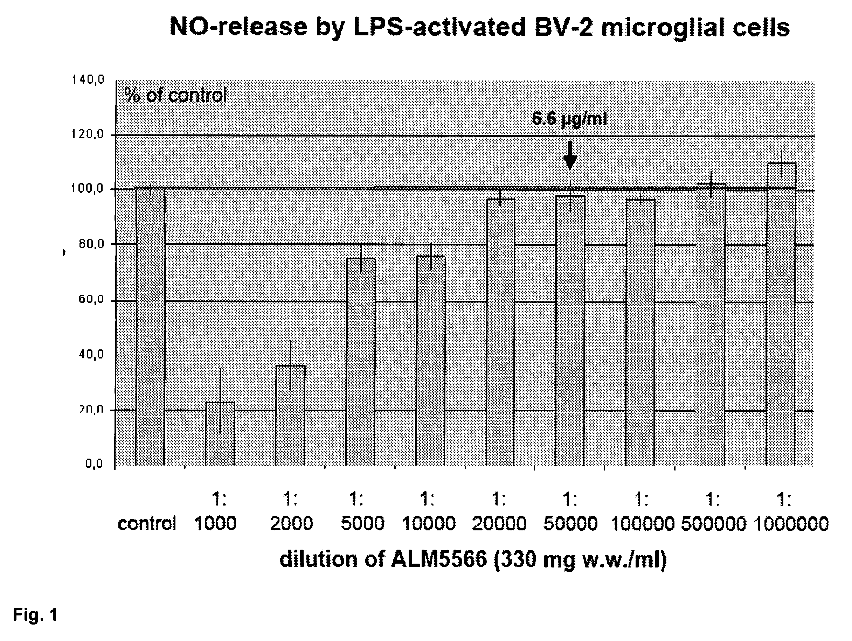

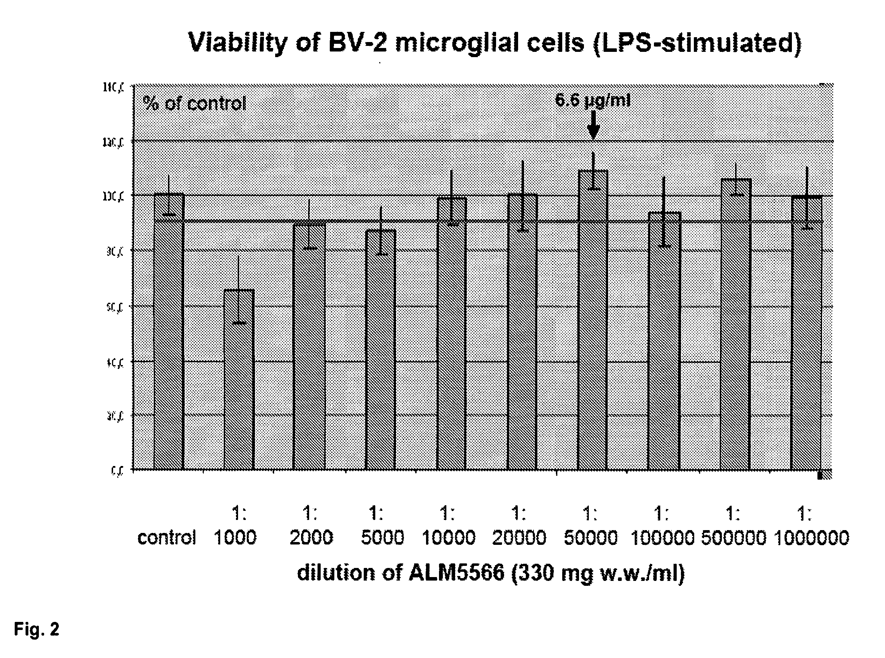

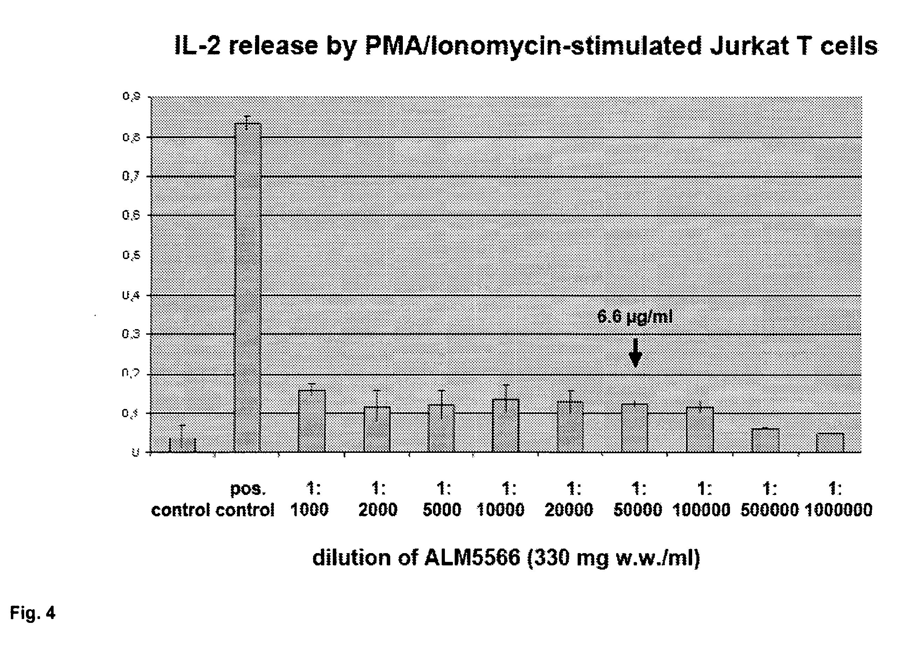

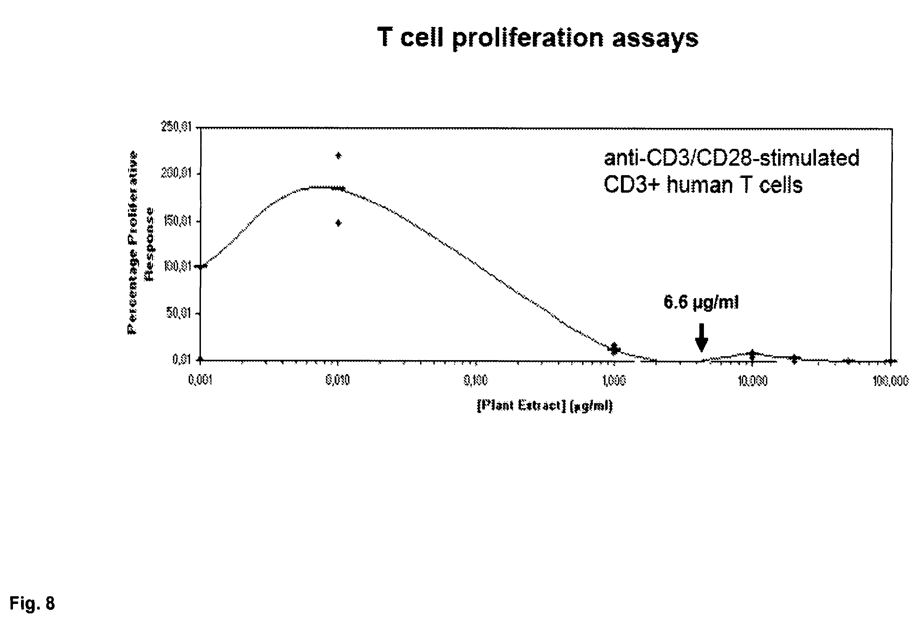

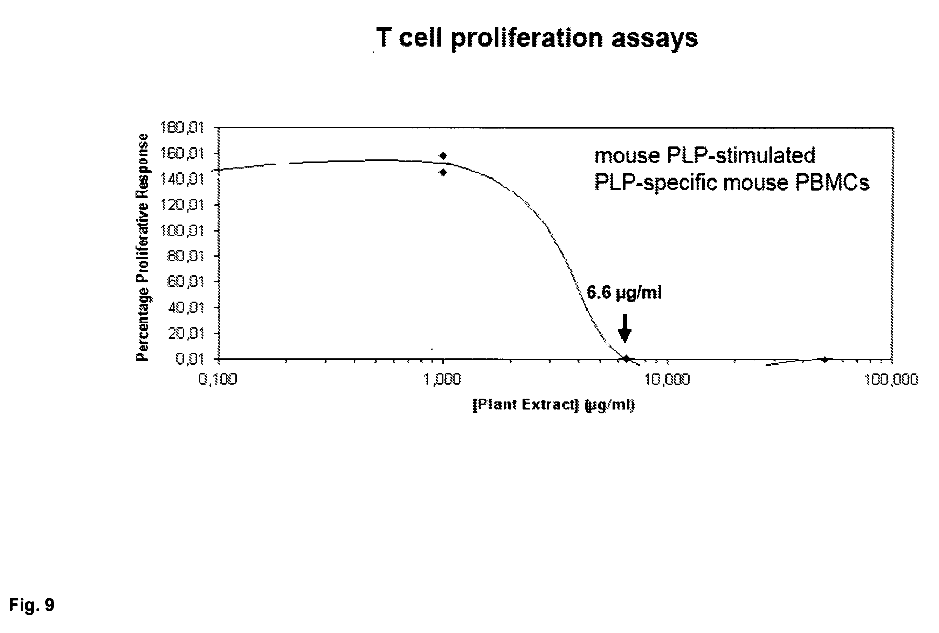

Surprisingly it was found that extracts of the invention inhibit the release of the proinflammatory cytokine IL-2 from activated T-cells without altering their viability (see Fig. 4), inhibit the proliferation rate of naïve and antigen-specific T-cells (see Fig. 8 and 9), inhibit the release of nitrogen monoxide (NO) from activated microglial cells without altering their viability (see Fig. 1 and 2), increase the viability of glutamate damaged neurons, and delay the onset of encephalomyelitis in an EAE mouse model while being well tolerated in these animals (see Fig. 16).

Interestingly, the extracts of the present invention were found to suppress signal transduction from the T cell receptor through the ras/raf/MAPK pathway and to disturb severely the assembly and phosphorylation state of membrane proximal signalosome complexes. Furthermore, the extracts of the invention suppress signal transduction through the ras/raf/MAPK pathway also after bypass of the membrane proximal compartment by PMA and the extracts lead to a severe disruption of cdc2 expression in primary human T cells. In summary the extracts of the invention posses anti-inflammatory and neuroprotective properties.

In a further aspect of the invention, the extract of the invention is used for the treatment or prophylaxis of a disease. In particular the extract or the pharmaceutical composition of the invention is used in the prophylaxis or treatment of an inflammatory and/or neurological disease. In a preferred embodiment, the extract or the pharmaceutical composition of the invention is used in the prophylaxis or treatment of autoimmune disease and/or transplant rejection.

In the sense of the present invention, the term "inflammatory disease" encompasses diseases that result from or are accompanied by unwanted reactions of the immune system. Such diseases can occur everywhere in the body. Inflammatory diseases comprise, but are not limited to, autoimmune diseases and transplant rejection. Examples of preferred inflammatory diseases are atherosclerosis, allergy, rheumatoid arthritis, Reiters syndrome, Morbus Crohn, Whipple disease, colitis ulcerosa, and systemic lupus erythematosus.

In the sense of the present invention, the term "neurologic disease" encompasses diseases that result from or are accompanied by damage and/or death of neuronal cells and where primary and secondary inflammatory processes play a role. Among these are diseases selected from e.g. Alzheimer's disease, Parkinson disease, amyotropic lateral sclerosis, craniocerebral injury, morbus Huntington, multiple sclerosis, stroke, encephalopathy, Creutzfeld-Jakob disease, and neurodegenerative disorders of the peripheral nervous system like e.g. polyneuropathy and polyneuritis.

For the avoidance of doubt, references herein to "treatment" include references to curative, palliative and prophylactic treatment.

According to another embodiment of the present invention, the extracts or the pharmaceutical compositions of the invention can also be used as a combination with one or more additional therapeutic agents to be co-administered to a patient to obtain some particularly desired therapeutic end result. The second and more additional therapeutic agents may also be an extract of the invention or a pharmaceutical composition thereof. More typically, the second and more therapeutic agents will be selected from a different class of therapeutic agents. As used herein, the terms "coadministration", "co-administered" and "in combination with", referring to the extracts of the invention and one or more other therapeutic agents, is intended to mean, and does refer to and include the following:
- simultaneous administration of such combination of extract(s) of the invention and therapeutic agent(s) to a patient in need of treatment, when such components are formulated together into a single dosage form which releases said components at substantially the same time to said patient,
- substantially simultaneous administration of such combination of extract(s) of the invention and therapeutic agent(s) to a patient in need of treatment, when such components are formulated apart from each other into separate dosage forms which are taken at substantially the same time by said patient, whereupon said components are released at substantially the same time to said patient,
- sequential administration of such combination of extract(s) of the invention and therapeutic agent(s) to a patient in need of treatment, when such components are formulated apart from each other into separate dosage forms which are taken at consecutive times by said patient with a significant time interval between each administration, whereupon said components are released at substantially different times to said patient; and
- sequential administration of such combination of extract(s) of the invention and therapeutic agent(s) to a patient in need of treatment, when such components are formulated together into a single dosage form which releases said components in a controlled manner whereupon they are concurrently, consecutively, and/or overlapingly administered at the same and/or different times by said patient, where each part may be administered by either the same or different route.

In a further aspect of the present invention, a pharmaceutical composition is provided comprising at least one extract from aerial parts of the plant *Hornstedtia scyphifera*.

In a preferred embodiment the pharmaceutical composition further comprises at least one pharmaceutically acceptable excipient. The term 'excipient' is used herein to describe any ingredient other than the extract(s) and ingredients of the extract of the invention. The choice of excipient will to a large extent depend on factors such as the particular mode of administration, the effect of the excipient on solubility and stability, and the nature of the dosage form.

In another preferred embodiment, the pharmaceutical composition further comprises at least one compound selected from the group containing saponins, flavone and derivatives thereof, tannins, sterols, proteins, carbohydrates, phenolic acids, xanthone and derivatives thereof, carotinoids and/or tri-terpenes.

The pharmaceutical composition according to this invention may further comprise an additional active ingredient or drug. This active ingredient or drug is present in addition to the extract of the invention. The extracts may be administered alone or in combination with one or more other extracts from *Hornstedtia scyphifera* of the invention or in combination with one or more other drugs (or as any combination thereof).

In the following the pharmaceutical compositions of the invention are described in more detail.

Pharmaceutical compositions suitable for the delivery of extracts of the present invention and methods for their preparation will be readily apparent to those skilled in the art. Such compositions and methods for their preparation may be found, for example, in Remington's Pharmaceutical Sciences. 19th Edition (Mack Publishing Company, 1995).

The extracts of the invention may be administered orally. Oral administration may involve swallowing, so that the extract enters the gastrointestinal tract, or buccal or sublingual administration may be employed by which the extract enters the blood stream directly from the mouth. Formulations suitable for oral administration include, but are not limited to, solid formulations such as tablets, capsules containing particulates, liquids, or powders, lozenges (including liquid-filled), chews, multi- and nano- particulates, gels, solid solution, liposome, films, ovules, sprays and liquid formulations.

Liquid formulations include suspensions, solutions, syrups and elixirs. Such formulations may be employed as fillers in soft or hard capsules and typically comprise a carrier, for example, water, ethanol, polyethylene glycol, propylene glycol, methylcellulose, or a suitable oil, and one or more emulsifying agents and/or suspending agents. Liquid formulations may also be prepared by the reconstitution of a solid.

The extracts of the invention may also be used in fast-dissolving, fast-disintegrating dosage forms such as those described in Expert Opinion in Therapeutic Patents, [upsilon] (6), 981-986, by Liang and Chen (2001).

For tablet dosage forms, depending on dose, the extract may make up from 1 weight % to 80 weight % of the dosage form, more typically from 5 weight % to 60 weight % of the dosage form. In addition to the extract, tablets generally contain a disintegrant. Examples of disintegrants include sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkylsubstituted hydroxypropyl cellulose, starch, pregelatinised starch and sodium alginate. Generally, the disintegrant will comprise from 1 weight % to 25 weight %, preferably from 5 weight % to 20 weight % of the dosage form.

Binders are generally used to impart cohesive qualities to a tablet formulation. Suitable binders include microcrystalline cellulose, gelatin, sugars, polyethylene glycol, natural and synthetic gums, polyvinylpyrrolidone, pregelatinised starch, hydroxypropyl cellulose and hydroxypropyl methylcellulose. Tablets may also contain diluents, such as lactose (monohydrate, spray-dried monohydrate, anhydrous and the like), mannitol, xylitol, dextrose, sucrose, sorbitol, microcrystalline cellulose, starch and dibasic calcium phosphate dihydrate.

Tablets may also optionally comprise surface active agents, such as sodium lauryl sulfate and polysorbate 80, and glidants such as silicon dioxide and talc. When present, surface active agents may comprise from 0.2 weight % to 5 weight % of the tablet, and glidants may comprise from 0.2 weight % to 1 weight % of the tablet.

Tablets also generally contain lubricants such as magnesium stearate, calcium stearate, zinc stearate, sodium stearyl fumarate, and mixtures of magnesium stearate with sodium lauryl sulphate. Lubricants generally comprise from 0.25 weight % to 10 weight %, preferably from 0.5 weight % to 3 weight % of the tablet.

Other possible ingredients include anti-oxidants, colorants, flavouring agents, preservatives and taste- masking agents. Exemplary tablets contain up to about 80 weight % extract, from about 10 weight % to about 90 weight % binder, from about 0 weight % to about 85 weight % diluent, from about 2 weight % to about 10 weight % disintegrant, and from about 0.25 weight % to about 10 weight % lubricant.

Tablet blends may be compressed directly or by roller to form tablets. Tablet blends or portions of blends may alternatively be wet-, dry-, or melt-granulated, melt congealed, or extruded before tabletting. The final formulation may comprise one or more layers and may be coated or uncoated; it may even be encapsulated.

The formulation of tablets is discussed in Pharmaceutical Dosage Forms: Tablets, Vol. 1 , by H. Lieberman and L. Lachman (Marcel Dekker, New York, 1980).

Consumable oral films for human or veterinary use are typically pliable water-soluble or water-swellable thin film dosage forms which may be rapidly dissolving or mucoadhesive and typically comprise an extract of the invention, a film-forming polymer, a binder, a solvent, a humectant, a plasticiser, a stabiliser or emulsifier, a viscosity-modifying agent and a solvent. Some components of the formulation may perform more than one function.

The extract of the invention may be water-soluble or insoluble. A water-soluble extract typically comprises from 1 weight % to 80 weight %, more typically from 20 weight % to 50 weight %, of the solutes. Less soluble extracts may comprise a greater proportion of the composition, typically up to 88 weight % of the solutes. Alternatively, the extracts of the invention may be in the form of multiparticulate beads.

The film-forming polymer may be selected from natural polysaccharides, proteins, or synthetic hydrocolloids and is typically present in the range 0.01 to 99 weight %, more typically in the range 30 to 80 weight %.

Other possible ingredients include anti-oxidants, colorants, flavourings and flavour enhancers, preservatives, salivary stimulating agents, cooling agents, co-solvents (including oils), emollients, bulking agents, anti-foaming agents, surfactants and taste-masking agents.

Films in accordance with the invention are typically prepared by evaporative drying of thin aqueous films coated onto a peelable backing support or paper. This may be done in a drying oven or tunnel, typically a combined coater dryer, or by freeze-drying or vacuuming.

Solid formulations for oral administration may be formulated to be immediate and/or modified release. Modified release formulations include delayed-, sustained-, pulsed- , controlled-, targeted and programmed release. Suitable modified release formulations for the purposes of the invention are described in US Patent No. 6,106,864. Details of other suitable release technologies such as high energy dispersions and osmotic and coated particles are to be found in Pharmaceutical Technology On-line. 25(2), 1-14, by Verma ef a/ (2001). The use of chewing gum to achieve controlled release is described in WO 00/35298.

The extracts of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications, they may be more suitably formulated as a sterile nonaqueous solution or as a dried form to be used in conjunction with a suitable vehicle such as sterile, pyrogen-free water.

The preparation of parenteral formulations under sterile conditions, for example, by lyophilisation, may readily be accomplished using standard pharmaceutical techniques well known to those skilled in the art.

The solubility of extracts of the invention used in the preparation of parenteral solutions may be increased by the use of appropriate formulation techniques, such as the incorporation of solubility- enhancing agents.

Formulations for parenteral administration may be formulated to be immediate and/or modified release. Modified release formulations include delayed-, sustained-, pulsed- , controlled-, targeted and programmed release. Thus compounds of the invention may be formulated as a solid, semi-solid, or thixotropic liquid for administration as an implanted depot providing modified release of the active compound. Examples of such formulations include drug-coated stents and poly(d/-Iactic-coglycolic)acid (PGLA) microspheres.

The extracts of the invention may also be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibres, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated - see, for example, J Pharm Sci, 88 (10), 955-958, by Finnin and Morgan (October 1999).

Other means of topical administration include delivery by electroporation, iontophoresis, phonophoresis, sonophoresis and microneedle or needle-free (e.g. Powderject(TM), Bioject(TM), etc.) injection. Formulations for topical administration may be formulated to be immediate and/or modified release. Modified release formulations include delayed-, sustained-, pulsed-, controlled-, targeted and programmed release.

The extracts of the invention can also be administered intranasally or by inhalation, typically in the form of a dry powder (either alone, as a mixture, for example, in a dry blend with lactose, or as a mixed component particle, for example, mixed with phospholipids, such as phosphatidylcholine) from a dry powder inhaler or as an aerosol spray from a pressurised container, pump, spray, atomiser (preferably an atomiser using electrohydrodynamics to produce a fine mist), or nebuliser, with or without the use of a suitable propellant, such as 1 ,1 ,1 ,2-tetrafluoroethane or 1 ,1 ,1,2,3,3,3-heptafluoropropane. For intranasal use, the powder may comprise a bioadhesive agent, for example, chitosan or cyclodextrin.

The pressurised container, pump, spray, atomizer, or nebuliser contains a solution or suspension of the compound(s) of the invention comprising, for example, ethanol, aqueous ethanol, or a suitable alternative agent for dispersing, solubilising, or extending release of the active, a propellant(s) as solvent and an optional surfactant, such as sorbitan trioleate, oleic acid, or an oligolactic acid.

Prior to use in a dry powder or suspension formulation, the drug product is micronised to a size suitable for delivery by inhalation (typically less than 5 microns). This may be achieved by any appropriate comminuting method, such as spiral jet milling, fluid bed jet milling, supercritical fluid processing to(form nanoparticles, high pressure homogenisation, or spray drying.

Capsules (made, for example, from gelatin or hydroxypropyl-methylcellulose), blisters and cartridges for use in an inhaler or insufflator may be formulated to contain a powder mix of the compound of the invention, a suitable powder base such as lactose or starch and a performance modifier such as /-Ieucine, mannitol, or magnesium stearate. The lactose may be anhydrous or in the form of the monohydrate, preferably the latter. Other suitable excipients include dextran, glucose, maltose, sorbitol, xylitol, fructose, sucrose and trehalose.

A suitable solution formulation for use in an atomiser using electrohydrodynamics to produce a fine mist may contain from 1 µg to 20mg of the extract of the invention per actuation and the actuation volume may vary from 1 µl to 100µl. A typical formulation may comprise an extract of the invention, propylene glycol, sterile water, ethanol and sodium chloride. Alternative solvents which may be used instead of propylene glycol include glycerol and polyethylene glycol.

Suitable flavours, such as menthol and levomenthol, or sweeteners, such as saccharin or saccharin sodium, may be added to those formulations of the invention intended for inhaled/intranasal administration. Formulations for inhaled/intranasal administration may be formulated to be immediate and/or modified release using, for example, PGLA. Modified release formulations include delayed-, sustained-, pulsed-, controlled-, targeted and programmed release.

In the case of dry powder inhalers and aerosols, the dosage unit is determined by means of a valve which delivers a metered amount or the drug product is packaged as discrete single dose units for use in the inhaler device. Units in accordance with the invention are typically arranged to administer a metered dose or "puff" containing from 1 µg to 4000 µg of the extract. The overall daily dose will typically be in the range 1 µg to 20 mg which may be administered in a single dose or, more usually, as divided doses throughout the day.

The extracts of the invention may be administered rectally or vaginally, for example, in the form of a suppository, pessary, or enema. Cocoa butter is a traditional suppository base, but various alternatives may be used as appropriate.

Formulations for rectal/vaginal administration may be formulated to be immediate and/or modified release. Modified release formulations include delayed-, sustained-, pulsed-, controlled-, targeted and programmed release.

The extracts of the invention may also be administered directly to the eye or ear, typically in the form of drops of a micronised suspension or solution in isotonic, pH-adjusted, sterile saline. Other formulations suitable for ocular and aural administration include ointments, biodegradable (e.g. absorbable gel sponges, collagen) and non-biodegradable (e.g. silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethyl cellulose, hydroxyethylcellulose, or methyl cellulose, or a heteropolysaccharide polymer, for example, gelan gum, may be incorporated together with a preservative, such as benzalkonium chloride. Such formulations may also be delivered by iontophoresis.

Formulations for ocular/aural administration may be formulated to be immediate and/or modified release. Modified release formulations include delayed-, sustained-, pulsed-, controlled-, targeted, or programmed release.

The extracts of the invention may be combined with soluble macromolecular entities, such as cyclodextrin and suitable derivatives thereof or polyethylene giycol-containing polymers, in order to improve their solubility, dissolution rate, taste-masking, bioavailability and/or stability for use in any of the aforementioned modes of administration.

Drug-cyclodextrin complexes, for example, are found to be generally useful for most dosage forms and administration routes. Both inclusion and non-inclusion complexes may be used. As an alternative to direct complexation with the drug, the cyclodextrin may be used as an auxiliary additive, i.e. as a carrier, diluent, or solubiliser. Most commonly used for these purposes are alpha-, beta- and gamma- cyclodextrins, examples of which may be found in International Patent Applications Nos. WO 91/11172, WO 94/02518 and WO 98/55148.

Inasmuch as it may be desirable to administer a combination of active compounds, for example, for the purpose of treating a particular disease or condition, it is within the scope of the present invention that one or more pharmaceutical compositions, preferably two or more pharmaceutical compositions, at least one of which contains an extract in accordance with the invention, may conveniently be combined in the form of a kit suitable for coadministration of the compositions.

Thus the kit of the invention comprises one or more separate pharmaceutical compositions, at least one of which contains an extract in accordance with the invention, and means for separately retaining said compositions, such as a container, divided bottle, or divided foil packet. An example of such a kit is the familiar blister pack used for the packaging of tablets, capsules and the like.

The kit of the invention is particularly suitable for administering different dosage forms, for example, oral and parenteral, for administering the separate compositions at different dosage intervals, or for titrating the separate compositions against one another. To assist compliance, the kit typically comprises directions for administration and may be provided with a so-called memory aid.

For administration to human patients, the total daily dose of the extracts of the invention is typically in the range 0.001 mg to 2000 mg depending, of course, on the mode of administration. For example, oral administration may require a total daily dose of from 0.1 mg to 2000 mg, while an intravenous dose may only require from 0.01 mg to 100 mg. The total daily dose may be administered in single or divided doses and may, at the physician's discretion, fall outside of the typical range given herein.

These dosages are based on an average human subject having a weight of about 60 kg to 70 kg. The physician will readily be able to determine doses for subjects whose weight falls outside this range, such as infants and the elderly.

In a further aspect of the invention the extract is used as active ingredient of a medicament.

The extract of the invention is used as dietary supplement alone or in combination with suitable additives and/or carriers. The skilled person is aware of such suitable additives and/or carriers and combinations thereof.

In the following, the invention is further described by figures and examples. These figures and examples are provided by way of illustration only and not by way of limitation.

### Figures

- Fig. 1: NO-release by LPS-activated BV-2 microglial cells. The release of NO from LPS-activated BV-2 microglial cells that were treated with various concentrations of the extract of the invention (ALM5566) is shown as percent of untreated control.
- Fig. 2: Viability of BV-2 microglial cells (LPS-stimulated). The viability of LPS-activated BV-2 microglial cells that were treated with various concentrations of the extract of the invention (ALM5566) is shown as percent of untreated control.
- Fig. 3: Viabiliity of BV-2 microglial cells (non-stimulated). The viability of non-LPS stimulated BV-2 microglial cells that were treated with various concentrations of the extract of the invention (ALM5566) is shown as percent of untreated control.
- Fig. 4: IL-2 release by PMA/lonomycin-stimulated Jurkat T cells. The release of the cytokine IL-2 from PMA/lonomycin-stimulated Jurkat T cells that were treated with various concentrations of the extract of the invention (ALM5566) is shown in comparison to stimulated and unstimulated control.
- Fig. 5: Viability of Jurkat T cells. The viability of Jurkat T cells that were treated with various concentrations of the extract of the invention (ALM5566) is shown as percent of untreated control. In addition a negative control is included that contains solvent in PBS without any extract.
- Fig. 6: Viability of HT22 neurons. The viability of HT22 neuronal cells that were treated with various concentrations of the extract of the invention (ALM5566) is shown in comparison to untreated control. In addition a negative control is included that contains solvent in PBS without any extract.
- Fig. 7: Viability of glutamate-damaged HT22 neurons. The viability of glutamate-damaged HT22 neuronal cells that were treated with various concentrations of the extract of the invention (ALM5566) is shown in comparison to untreated control. In addition a negative control is included that contains solvent in PBS without any extract.
- Fig. 8: T cell proliferation assay (naïve). The percentage proliferative response of anti-CD3/CD28-stimulated CD3+ human T cells (naïve) that were treated with various concentrations of the extract of the invention (ALM5566) is shown.
- Fig.9: T cell proliferation assay (antigen specific)). The percentage proliferative response of mouse PLP-stimulated PLP-specific mouse PBMCs (peripheral blood mononuclear cells) that were treated with various concentrations of the extract of the invention (ALM5566) is shown.
- Fig.10: Jurkat T cells: Cytosolic signal transduction after stimulation of T cell receptor. Shown is a western blot result for members of the ras/raf/MAPK signalling pathway and their phosphorylation state upon stimulation of the CD3/TCR of Jurkat T cells with conA depending on the presence or absence of the extract of the invention (ALM5566) over time.
- Fig.11: Jurkat T cells: Cytosolic signal transduction after bypass of membrane proximal compartment by PMA. Shown is a western blot result for members of the ras/raf/MAPK signalling pathway and their phosphorylation state upon treatment of the CD3/TCR of Jurkat T cells with PMA depending on the presence or absence of the extract of the invention (ALM5566) over time.
- Fig.12: Jurkat T cells: Membrane proximal signal transduction after stimulation of T cell receptors. Shown is a western blot result for members of the membrane associated signalosome of CD3/TCR and their phosphorylation state upon activation of CD3/TCR of Jurkat T cells with conA depending on the presence or absence of the extract of the invention (ALM5566).
- Fig.13: Cell cycle regulation in human T cells. This figure shows a western blot result for members of cell cycle regulation pathway in human T cells and their phosphorylation states upon activation with conA and treated with various concentrations of the extract of the invention (ALM5566).
- Fig.14: This figure shows the proliferative response of lymphocytes isolated from spleen of SJL mice after 10 days of treatment with various concentrations of the extract (ALM5566). A significance value of p < 0,05 is marked by an asterix.
- Fig.15: Active EAE in SJL mice daily treated with 1mg extract (ALM5566) per mouse. This figure shows the mean clinical score of untreated SJL mice and SJL mice treated daily with 1mg ALM5566 per mouse over time. Significant differences between the two groups are marked with an asterix.

### Examples

### 1. Preparation of extract from the plant Hornstedtia scyphifera

Parts (leafs, bark, root) of the plant *Hornstedtia scyphifera* have been collected from Frasers Hill, western Malaysia; Danum Valley, Sabah; and/or Kelabit Highlands, Sarawak. 10 to 20 leafs (20-1 00g) and bark and/or root material with a weight of 100-200g were dried, powdered and extracted with methanol for two days at room temperature. The pith was removed by filtration and the solvent was eliminated by rotovaporation (Buchi, Schweiz). The crude methanol extract was partitioned with a 1:1 mixture of chloroform and water. The undissolved material was removed by filtration and the solvent was separated by a separation funnel. The aqueous extract was freeze-dried. The remaining chloroform extract was freed from the solvent by evaporation. The chloroform extract was partitioned further with a 1:1 mixture of hexan and 20% aqueous methanol. Again the undissolved material was removed by filtration. The filtered remainder was separated by a separating funnel in a hexan extract and an aqueous methanol extract.

This methanol extract was shipped from the National University of Malaysia, Bangi, Kuala Lumpur, Malaysia to the Institute of Cell- and Neurobiology, Center of Anatomy (formerly Institute of Anatomy), Universitätsmedizin Berlin. There, the methanolic extract was dried to the maximum under nitrogen atmosphere at room temperature and the dry weight of the extract was determined. Subsequently the dried extract was resolved in 5ml ethanol. Different dilutions were prepared in PBS and stored at -80°C.

In the subsequent experiments, the extract was used in dilutions of the resolved extract in PBS ranging from 10⁻⁶ to 10⁻³. In each test, every dilution was analysed in 8 independent experiments. In all experiments described below, samples comprising the extract of the invention are termed ALM5566.

### 2. NO-release by LPS-activated BV-2 microglial cells

This test is designed to reveal the effect of the extract (ALM5566) on the immuno-effector function of BV-2 microglial cells. The NO-release of BV-2 cells is measured in response to stimulation of the CD14/TLR4 receptor with LPS (lipopolysaccharide). This test has been chosen, because NO-release by microglial cells is common to pathologically overactive microglial cells in a number of neurological and/or inflammatory diseases.

BV-2 cells are primary mouse microglial cells immortalized by stable transfection with the c-myc-oncogene using a J7-retrovirus (Blasi, E., Barluzzi, R., Bocchini, V., Mazzolla, R., and Bistoni, F. (1990) Immortalization of murine microglial cells by a v-raf/v-myc carrying retrovirus. J Neuroimmunol 27, 229-237), leading to a phenotype functionally identical to native primary microglia and well adapted for serving as a model system (Laurenzi, M. A., Arcuri, C., Rossi, R., Marconi, P., and Bocchini, V. (2001) Effects of microenvironment on morphology and function of the microglial cell line BV-2. Neurochem Res 26, 1209-1216). After activation they show the typical amoeboid morphology and are capable of phagocytosis (Bocchini, V., Mazzolla, R., Barluzzi, R., Blasi, E., Sick, P., and Kettenmann, H. (1992) An immortalized cell line expresses properties of activated microglial cells. J Neurosci Res 31, 616-621). BV-2 microglia cells were cultured in Dulbecco's modified Eagle's medium (DMEM) supplemented with FCS (10%), glutamin (10%), penicillin/streptomycin (5%) at a density not exceeding 2 x 10⁵ cells/ml. Microglial cells were activated by with lipopolysaccharide (LPS from *E. coli,* Serotyp 0111:B4, Sigma-Aldrich, Germany) in a concentration of 10 µg/ml. For all cell-based assays BV-2 cells were seeded at day 0 in sterile 96-well-plates at a density of 5 x 10⁴cells/ml and left to recover to their resting state in the incubator (37°C, 5% CO₂, 95% air). 24h later (day 1) all supernatant was carefully discarded and a group specific combination of medium, extract dilution or control dilution, and LPS was added. Another 24h later (day 2) the BV-2 supernatant was removed and analysed. The adherent BV-2 cells remained in the well and were used for semiquantitative viability analysis via cell activity assays (see below) or were harvested with a cell scraper, washed, centrifuged and used for cellular or nucleic protein analysis.

Supernatants obtained from cell culture experiments were analysed quantitatively for stable nitrites derived from unstable nitric oxide in accordance with the method described by Green *et al*. (Green, L. C., Wagner, D. A., Glogowski, J., Skipper, P. L., Wishnok, J. S., and Tannenbaum, S. R. (1982) Analysis of nitrate, nitrite, and [15N]nitrate in biological fluids. Anal Biochem 126, 131-138) with Griess Reagent (Sulfanilamid [1% in H2O] 1:1 mixed with N-1-naphtylethylendiamin dihydrochloride [0.1 % in 5% phosphoric acid]) detected at an optical density of 540 nm.

In a parallel experiment, the effect of different extract dilutions on the viability of LPS-stimulated BV-2 microglial cells was measured in order to exclude that a potential inhibition in NO-release was an unspecific consequence of reduced cell viability.

Cell viability assays were performed via mitochondrial conversion of MTT (3-[4,5-dimethylthiazol-2-yl]-2,5-diphenyl tetrazolium bromid) and subsequent detection of optical density at 563 nm according to the method of Mosman et al. (Mosmann, T. (1983) Rapid colorimetric assay for cellular growth and survival: application to proliferation and cytotoxicity assays. J Immunol Methods 65, 55-63).

A dilution of the solvent in PBS without extract served as negative control in both tests in order to exclude that traces of ethanol or PBS may be responsible for the effects observed.

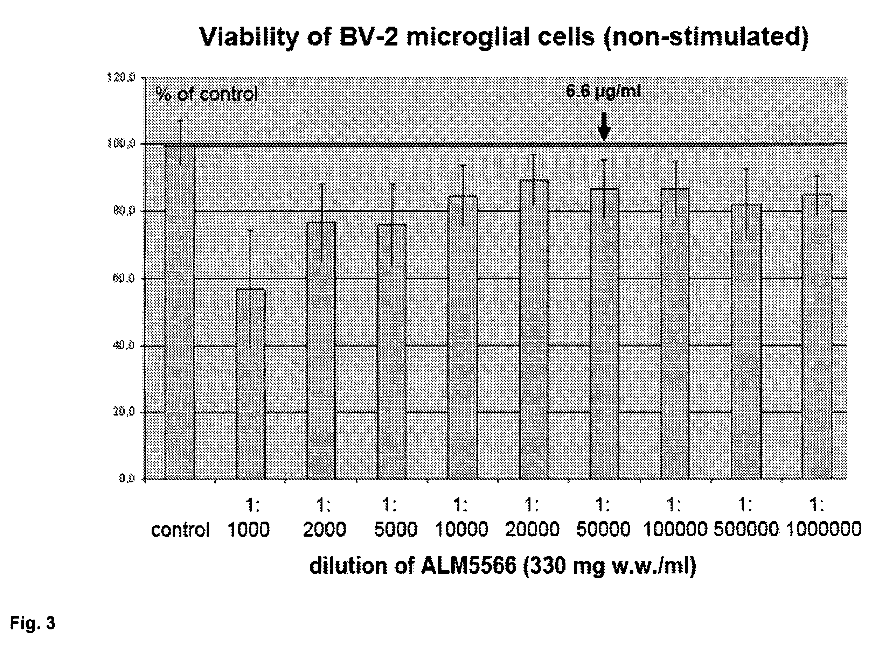

The test revealed that administration of the extract leads to a dose dependent inhibition of NO-release from the LPS-stimulated BV-2 microglial cells (Fig. 1), while the cell viability of the microglial cells was not significantly affected under the same conditions, irrespective whether the BBV-2 cells were LPS-stimulated or not (Fig. 2 and 3).

### 3. IL-2 release from PMA/lonomycin-stimulated Jurkat T cells

This test is designed to reveal the effect of the extract (ALM5566) on the ability of T cells to release the cytokine IL-2 upon stimulation with PMA/Ionomycin. IL-2 is well know as a proinflammatory and proliferation stimulating cytokine involved in a number of pathologic neurologic and/or inflammatory processes.

Supernatants from cell culture experiments were analysed for interleukin 2 (IL-2) using a IL-2 ELISA Set (BD Biosciences), which contained anti-IL-2 monoclonal antibody, biotinylated anti-IL-1 monoclonal antibody and avidin-horseradish peroxidase conjugate as enzyme reagent. Optical density was detected at 450 nm.

In a parallel experiment, the effect of different extract dilutions on the viability of PMA/Ionomycin-stimulated Jurkat T cells was measured in order to exclude that a potential inhibition in IL-2-release was an unspecific consequence of reduced cell viability.

Cell viability assays were performed via mitochondrial conversion of MTT (3-[4,5-dimethylthiazol-2-yl]-2,5-diphenyl tetrazolium bromid) and subsequent detection of optical density at 563 nm according to the method of Mosman et al. (Mosmann, T. (1983) Rapid colorimetric assay for cellular growth and survival: application to proliferation and cytotoxicity assays. J Immunol Methods 65, 55-63).

A dilution of the solvent in PBS without extract served as positive control in the IL-2 release test and as a negative control in the viability test in order to exclude that traces of ethanol or PBS may be responsible for the effects observed.

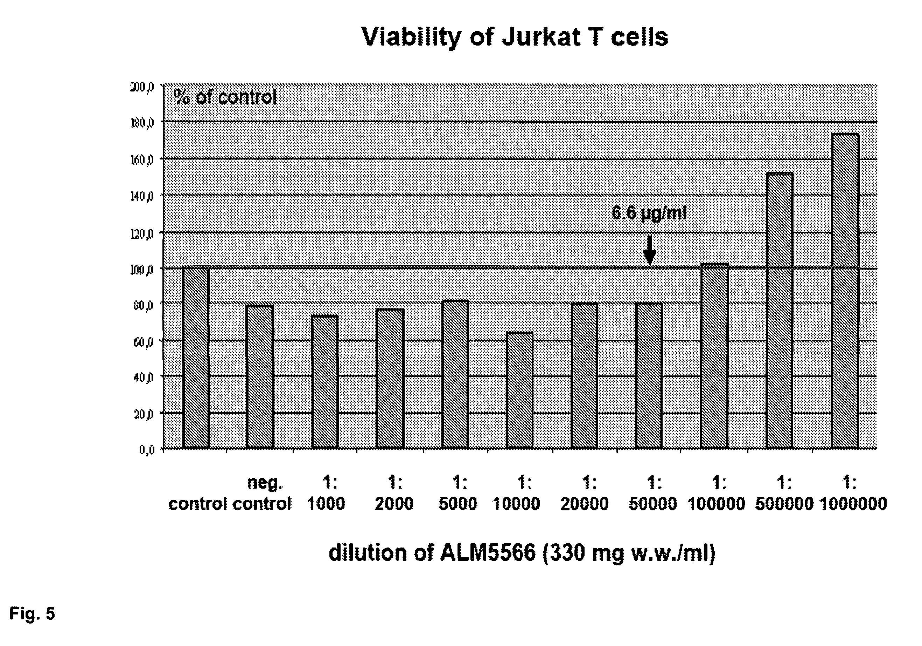

The results clearly show that the extract is capable of inhibiting IL-2 release from stimulated T cells already at very low concentrations (see Fig. 4), while the effect of the extract on T cell viability is not significant (Fig. 5).

### 4. Viability of glutamate-damaged HT22 neurons

In this test it was investigated whether the extract (ALM5566) of the invention has a neuroprotective effect on neuronal cells. For this purpose HT22 cells, deficient in NMDA receptor, were treated with glutamate in the absence or presence of different dilutions of the extract. Subsequently cell viability was measured.

HT-22 cells are a murine hippocampal cell line, which were developed from immortalized HT-4 cells (Maher, P., and Davis, J. B. (1996) The role of monoamine metabolism in oxidative glutamate toxicity. J Neurosci 16, 6394-6401). HT-4 cells have been generated from murine neuronal stem cells by McKay (McKay, R., Valtz, N., Cunningham, M., and Hayes, T. (1990) Mechanisms regulating cell number and type in the mammalian central nervous system. Cold Spring Harb Symp Quant Biol 55, 291-301) in accordance with the method described by Frederiksen *et al* (Frederiksen, K., Jat, P. S., Valtz, N., Levy, D., and McKay, R. (1988) Immortalization of precursor cells from the mammalian CNS. Neuron 1, 439-448*)*. HT-22 cells carry the metabotropic NMDA-glutamate-receptor, but not the ionotropic glutamate-receptor and they do not synthesize certain monoamines (norepinephrine, DOPA, dopamine, epinephrine). The cells are cultivated in DMEM medium supplemented with FCS (10%), glutamin (10%), penicillin/streptomycin (5%) and glucose. HT-22 were seeded (day 0) in sterile 96-well-plates at a density of 1 x 10⁵ cells/ml and left to recover and adhere for 24h in the incubator (37°C, 5% CO₂, 95% air). On the next day (day 1) all supernatant was carefully discarded and a group specific combination of medium, extract or control solution and either glutamate [20 mM], H₂O₂ [150 µM] or nitric oxide donator sodium nitroprussid [250 µM] was added to induce different modes of neuronal damage. One experimental group only contained medium combined with extract solution or control solution to assess extract specific neurotoxicity. All damaging substances were solved in sterile PBS. 24h later (day 2) HT-22 cell viability was assessed using cell viability assays. Non-damaged cells were set as 100% viability control and damaged untreated cells were set as 0% viability control. For controls, vehicle (ethanol dilutions in PBS according to the substance samples) were used.

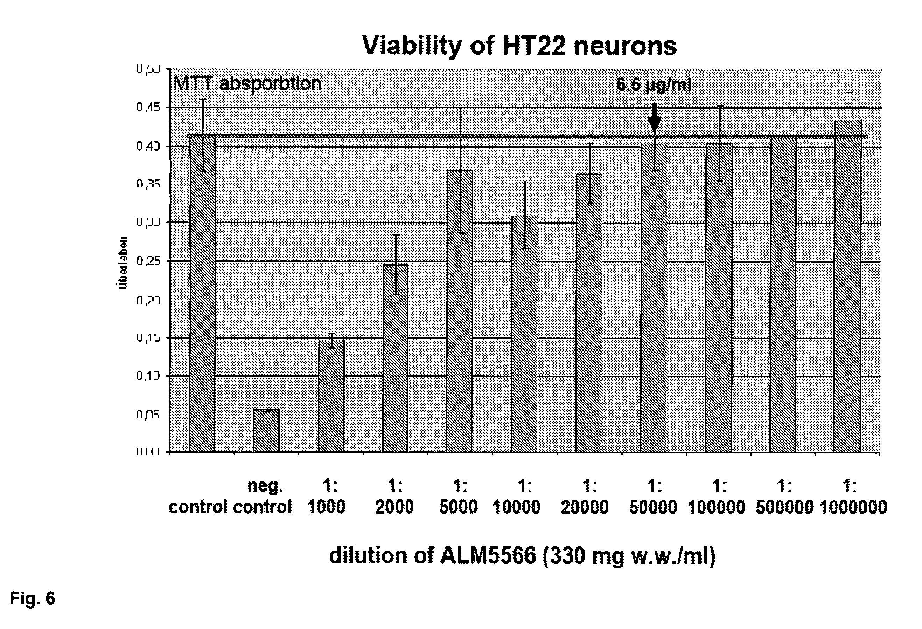

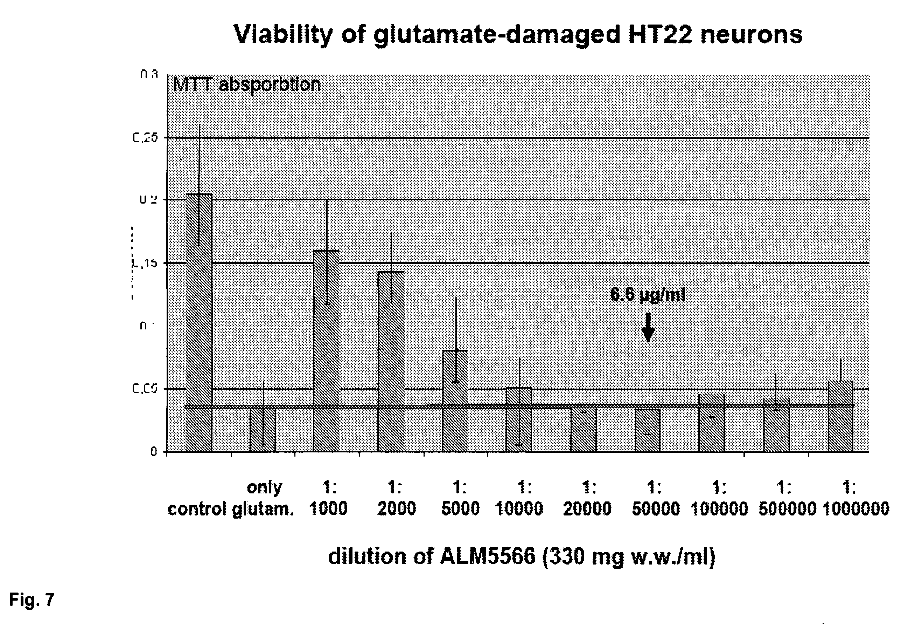

The results clearly demonstrate that while the extract has a dose dependent negative effect on cell viability of non-damaged HT22 neurons (Fig. 6), the extract is capable to protect dose dependently HT22 cells from a reduction in cell viability in response to glutamate damage (Fig. 7). The extract of the invention has neuroprotective properties.

### 5. Effect of the extract on T cells

### a) T cell proliferation assays

In this test it was investigated whether the extract (ALM5566) of the invention is able to influence the proliferation rate of cells of the immune system like T cells. Naïve human CD3 positive T cells were stimulated with anti-CD3/CD28 agents and proliferation of the cells.

In a second test antigen-specific (PLP-specific) mouse PBMCs were stimulated with mouse PLP and again proliferation of the stimulated cells in absence or presence of different dilutions of the extract of the invention was measured.

Human PBMC (peripheral immune cells) were obtained from heparinized peripheral blood of healthy donors in accordance to local ethics committee and isolated by Ficoll Hypaque density gradient centrifugation. Immunophenotypic analysis of PBMC, on a FACSCalibur (BD Biosciences), revealed CD45+ RA+ CD3+ T cells, which were polyclonaly stimulated with anti-CD3 and anti-CD28 antibodies. Further murine PBMC were stimulated with PLP139-151. 3H-thymidine incorporation assay was performed to determine the proliferation.

As shown in Fig. 8 and 9, the extract inhibited both the proliferation of naïve and antigen-specific T cells in a dose dependent manner.

### b) Signal transduction after stimulation of T cell receptor (CD3/TCR)

The signal transduction cascade triggered upon activation of the CD3/TCR involves the cytosolic ras/raf/MAPK pathway and the membrane associated Grb2/PLCγ1 pathway. In this test it was investigated whether treatment with the extract (ALM5566) of the invention has any influence on the T cell receptor signalling cascade within Jurkat T cells.

Membranes have been recovered by ultracentrifugation (40min, 33000rpm, 4°C), washed in PBS and transferred in lysis buffer containg 1% NP-40. *Lipid Rafts* were prepared according to Zhang (Zhang W, Trible RP, Samelson LE. LAT palmitoylation: its essential role in membrane microdomain targeting and tyrosine phosphorylation during T cell activation. Immunity 9(2), 239-246 (1998)): Briefly, T cells have been lysed in a Dounce-homogenisator containing ice-cold homogenisation buffer (25mM MES, pH6,5, 100mM NaCl, 5mM EDTA, 1% Triton X-100 with 1mM sodium orthovanadate, 1mM PMSF, 10mM sodium pyrophosphate, 50mM sodium fluoride), the lysate has been loaded on an 40-5% sucrose gradient and ultracentrifuged at 200000g for 20h. Fractions with 0.4ml have been collected from the surface of the gradient and lipid raft-fractions have been identified by immunoblots using a phospho-tyrosin-antibody. Phosphorylation of LAT and assembly of SLP-76, Gads, Itk and PLC- have been analysed by specific antibodies in immunoblots. Analysis of signal transduction pathways was performed by separating equal amounts of protein per sample with 12% polyacrylamid gel SDS-page-electrophoresis technique. Separated proteins were transblotted onto nitrocellulose membranes with Western Blot technique. Membranes were incubated with blocking buffer [20 mM Tris, pH 7.6; 140 mM NaCl; 0.05% Tween-20; 5% nonfat dry milk] prior to incubation with primary monoclonal antibodies diluted in blocking buffer. After washing 3 times in Tris-buffered saline containing 0.1 % Tween-20 (TBS-T), membranes were incubated with the appropriate horseradish peroxidase-conjugated secondary antibody. Membranes were subsequently washed 3 times in TBST and a chemiluminescence-based detection with luminol reagent was performed according to manufacturer's protocol (Amersham Biosciences).

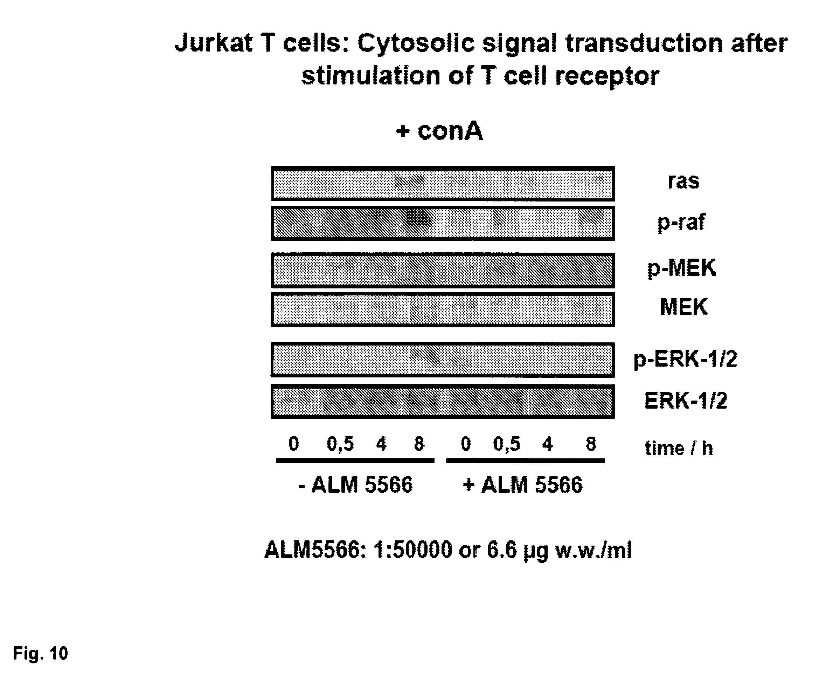

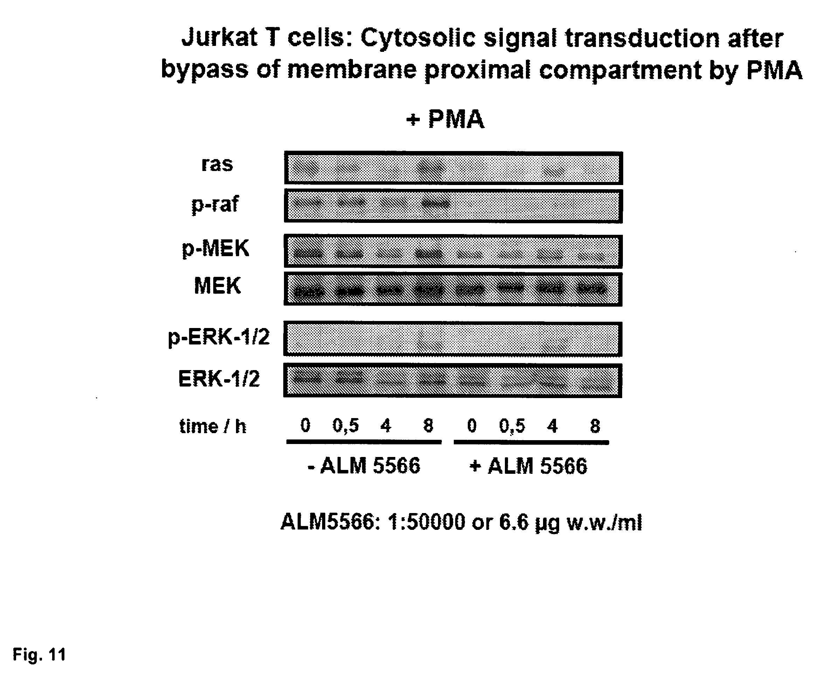

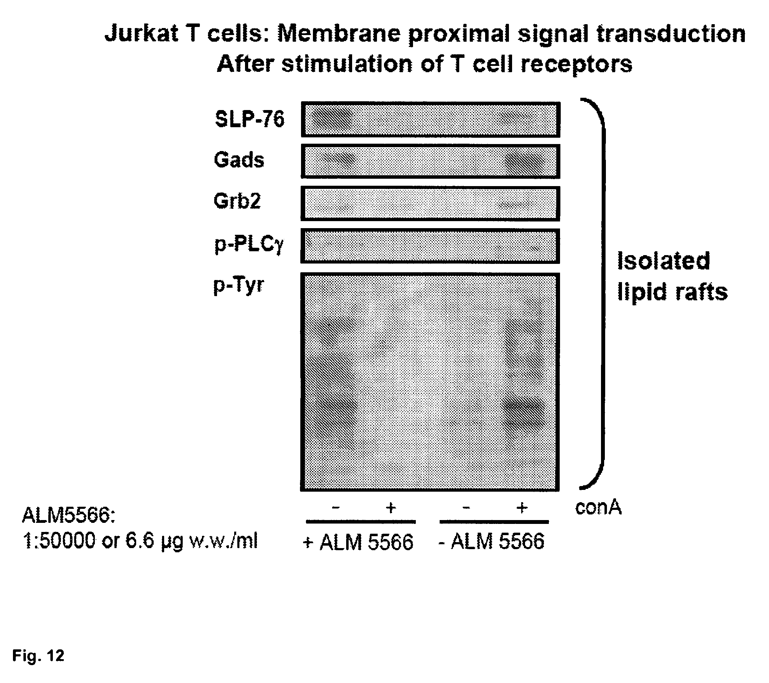

As shown in Fig. 10, the extract of the invention suppresses signal transduction from the T cell receptor through the ras/raf/MAPK pathway. Bypassing the membrane proximal compartment by PMA did not lead to another result, the ras/raf/MAPK pathway remains to be inhibited by the extract (see Fig. 11). In addition, the assembly and phosphorylation of the membrane proximal signalosome complex is severely disturbed in T cells treated with the extract (Fig. 12).

### c) Effect of extract on cell cycle regulation of human T cells

As shown above, the extract of the invention (ALM5566) is capable of inhibiting proliferation of T ells in a dose dependent manner. In this test, it was investigated whether this inhibitory effect was due to an influence on the regulation of the cell cycle.

Test was performed as described above.

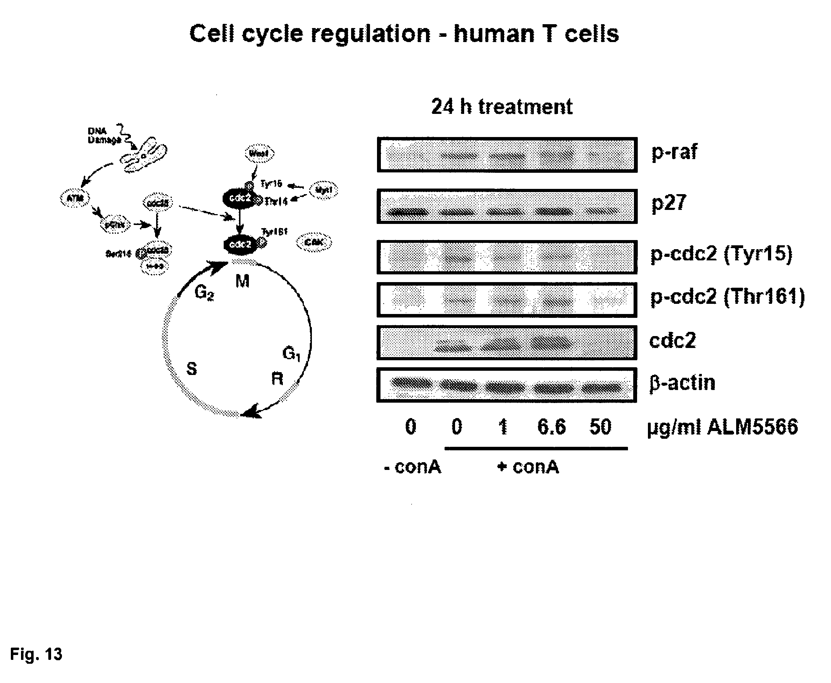

As shown in Fig. 13, primary human T cells treated with high concentrations of extract reveal a significant inhibition of cdc2 expression, an important molecule in the regulation cascade of T cell cycle.

### d) Proliferative response of lymphocytes isolated from SJL mice treated with extract

In the tests described above, the effect of the extract of the invention (ALM5566) on T cell proliferation and signal transduction was analysed *in vitro.* With this test, it was investigated whether the extract shows also an effect on T cells *in vivo*.

Spleen cells were derived from PLP139-151-immunized mice. T cell response was investigated after 10 days upon exposure to ConA.

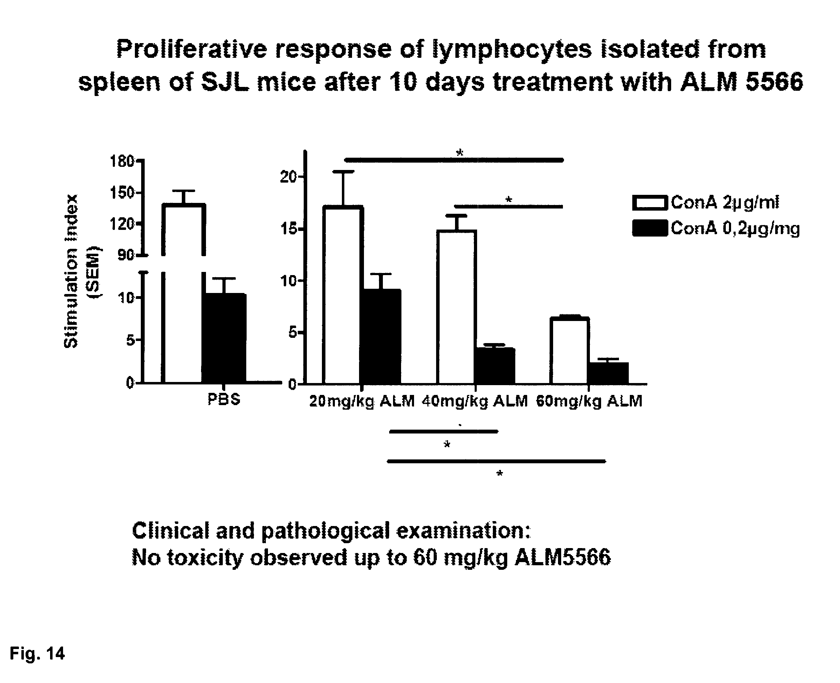

The results reveal that administration of the extract leads to an inhibition of the proliferative response of lymphocytes isolated from the spleen of SJL mice after 10 days of treatment with the extract. This inhibitory effect was dose dependent with higher inhibition rates at higher doses of the extract (Fig. 14). Clinical and pathological examination of the treated mice revealed no detectable toxicity of the extract up to the highest dose administered of 60 mg/kg extract (ALM5566).

### 6. Effect of the extract on EAE in SJL mice

In this test, it was analysed whether the administration of the extract of the invention (ALM5566) *in vivo* has any effect on experimental autoimmune encephalomyelitis (EAE) in SJL mice.

Female SJL mice (6-8 weeks; approximately 20 g body weight; Charles-River) were immunized s.c. with 75 µg proteolipid protein (PLP) 139-151 in 0.2 ml emulsion consisting of equal volumes of PBS and CFA and containing 6 mg/ml of Mycobacterium tuberculosis H37Ra. Pertussis toxin (200 ng) was administered i.p. at days 0 and 2. Mice were scored for EAE as follows: 0, no disease; 1, tail weakness; 2, paraparesis; 3, paraplegia; 4, paraplegia with forelimb weakness or paralysis; 5, moribund or dead animals. Mean clinical scores at separate days and mean maximal scores were calculated by adding scores of individual mice and dividing by number of mice in each group. All procedures were conducted according to protocols approved by the local animal welfare committee.

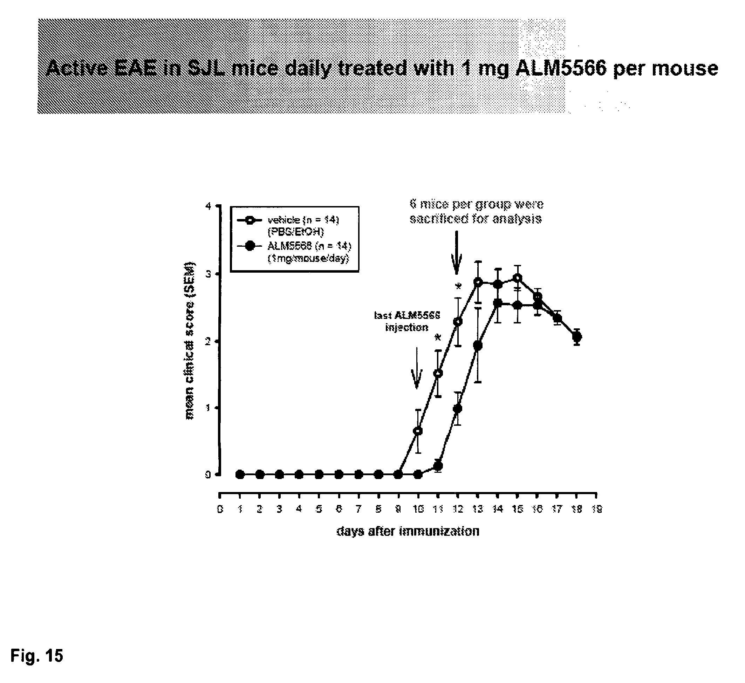

As shown in Fig. 15, the onset of clinically manifest EAE in mice treated with the extract was significantly delayed. Also the peak of the mean clinical score of EAE was lower in the treated group compared to the untreated control. The extract clearly showed a positive effect on an inflammatory and neurological disease like EAE.

## Claims

1. Extract from the plant *Hornstedtia scyphifera* obtainable by
- extracting leaf and bark and/or root material of *Homstedtia scyphifera* with methanol;
- partitioning the methanol extract with 1:1 mixture of chloroform and water;
- removing undissolved material;
- freeing chloroform extract from solvent and partitioning the extract with a 1:1 mixture of hexan and 20% aqueous methanol;
- removing undissolved material;
- separating the remainder in a hexan extract and an aqueous methanol extract;
- drying the methanolic extract and resolving the dried extract in ethanol.

2. Pharmaceutical composition comprising an extract according to claim 1.

3. Pharmaceutical composition of claim 2 further comprising at least one pharmaceutically acceptable excipient.

4. Pharmaceutical composition according to claim 2 or 3 further comprising at least one compound selected from the group containing saponins, flavone and derivatives thereof, tannins, sterols, proteins, carbohydrates, phenolic acids, xanthone and derivatives thereof, carotinoids and/or tri-terpenes.

5. Pharmaceutical composition according to anyone of claims 2 to 4 further comprising an additional active ingredient.

6. Kit comprising one or more separate pharmaceutical compositions, at least one of which contains an extract according to claim 1.

7. Method of manufacturing an extract of claim 1 comprising the step of:
- extracting leaf and bark and/or root material of *Homstedtia scyphifera* with methanol;
- partitioning the methanol extract with 1:1 mixture of chloroform and water;
- removing undissolved material;
- freeing chloroform extract from solvent and partitioning the extract with a 1:1 mixture of hexan and 20% aqueous methanol;
- removing undissolved material;
- separating the remainder in a hexan extract and an aqueous methanol extract;
- drying the methanolic extract and resolving the dried extract in ethanol

8. Extract of claim 1 for use as active ingredient of a medicament.

9. Extract of claim 1 for use in the treatment or prophylaxis of a disease.

10. Extract of claim 1 as dietary supplement.

11. Extract of claim 1 or a pharmaceutical composition of anyone of claims 2 to 5 for use in the prophylaxis or treatment of an inflammatory and/or neurological disease or as dietary supplement.

12. Extract of claim 1 or a pharmaceutical composition of anyone of claims 2 to 5 for use in the prophylaxis or treatment of autoimmune disease and/or transplant rejection.

13. Pharmaceutical composition of anyone of claims 11 to 12 for use in combination with one or more additional therapeutic agents.

## Patentansprüche

1. Extrakt aus der Pflanze *Hornstedtia scyphifera,* erhältlich durch:
- Extrahieren von Blatt- und Rinden- und/oder Wurzelmaterial von *Hornstedtia scyphifera* mit Methanol;
- Aufteilen des Methanolextrakts mit einem 1:1-Gemisch aus Chloroform und Wasser;
- Entfernen von ungelöstem Material;
- Befreien des Chloroform-Extrakts von Lösungsmittel und Aufteilen des Extrakts mit einem 1:1-Gemisch aus Hexan und 20 %-wasserhaltigem Methanol;
- Entfernen von ungelöstem Material;
- Trennen des Rückstands in einen Hexanextrakt und einen wasserhaltigen Methanolextrakt;
- Trocknen des methanolhaltigen Extrakts und Lösen des getrockneten Extrakts in Ethanol.

2. Pharmazeutische Zusammensetzung, umfassend einen Extrakt nach Anspruch 1.

3. Pharmazeutische Zusammensetzung nach Anspruch 2, ferner umfassend zumindest einen pharmazeutisch annehmbaren Hilfsstoff.

4. Pharmazeutische Zusammensetzung nach Anspruch 2 oder 3, ferner umfassend zumindest eine Verbindung, ausgewählt aus der Gruppe, enthaltend Saponine, Flavone und deren Derivative, Tannine, Sterine, Proteine, Kohlenhydrate, Phenolsäuren, Xanthon und dessen Derivate, Carotinoide und/oder Triterpene.

5. Pharmazeutische Zusammensetzung nach einem der Ansprüche 2 bis 4, ferner umfassend einen zusätzlichen Wirkstoff.

6. Kit, umfassend eine oder mehrere separate pharmazeutische Zusammensetzungen, wovon zumindest eine einen Extrakt nach Anspruch 1 enthält.

7. Verfahren zum Herstellen eines Extrakts nach Anspruch 1, umfassend folgende Schritte:
- Extrahieren von Blatt- und Rinden- und/oder Wurzelmaterial von Hornstedtia scyphifera mit Methanol;
- Aufteilen des Methanolextrakts mit einem 1:1-Gemisch aus Chloroform und Wasser;
- Entfernen von ungelöstem Material;
- Befreien des Chloroform-Extrakts von Lösungsmittel und Aufteilen des Extrakts mit einem 1:1-Gemisch aus Hexan und 20 %-wasserhaltigem Methanol;
- Entfernen von ungelöstem Material;
- Trennen des Rückstands in einen Hexanextrakt und einen wasserhaltigen Methanolextrakt;
- Trocknen des methanolhaltigen Extrakts und Lösen des getrockneten Extrakts in Ethanol.

8. Extrakt nach Anspruch 1 zur Verwendung als Wirkstoff eines Medikaments.

9. Extrakt nach Anspruch 1 zur Verwendung bei der Behandlung oder Prophylaxe einer Erkrankung.

10. Extrakt nach Anspruch 1 als Nahrungsergänzungsmittel.

11. Extrakt nach Anspruch 1 oder pharmazeutische Zusammensetzung nach einem der Ansprüche 2 bis 5 zur Verwendung bei der Prophylaxe oder Behandlung einer entzündlichen und/oder neurologischen Erkrankung oder als Nahrungsergänzungsmittel.

12. Extrakt nach Anspruch 1 oder pharmazeutische Zusammensetzung nach einem der Ansprüche 2 bis 5 zur Verwendung bei der Prophylaxe oder Behandlung einer Autoimmunerkrankung und/oder Transplantatabstoßung.

13. Pharmazeutische Zusammensetzung nach einem der Ansprüche 11 bis 12 zur Verwendung in Kombination mit einem oder mehreren zusätzlichen Therapeutika.

## Revendications

1. Extrait de la plante *Hornstedtia scyphifera,* pouvant être obtenu par :
- l'extraction de matière de feuilles et d'écorce et/ou de racines de *Hornstedtia scyphifera* avec du méthanol ;
- la séparation de l'extrait de méthanol avec un mélange 1:1 de chloroforme et d'eau ;
- l'enlèvement de la matière non dissoute ;
- la libération de l'extrait de chloroforme par rapport au solvant et la séparation de l'extrait avec un mélange 1:1 d'hexane et de méthanol aqueux à 20 % ;
- l'enlèvement de la matière non dissoute ;
- la division du reste en un extrait d'hexane et un extrait de méthanol aqueux ;
- le séchage de l'extrait méthanolique et la décomposition de l'extrait sec dans de l'éthanol.

2. Composition pharmaceutique comprenant un extrait selon la revendication 1.

3. Composition pharmaceutique selon la revendication 2, comprenant également au moins un excipient acceptable au plan pharmaceutique.

4. Composition pharmaceutique selon la revendication 2 ou 3, comprenant également au moins un composé sélectionné dans le groupe contenant des saponines, de la flavone et leurs dérivés, des tanins, des stérols, des protéines, des hydrates de carbone, des acides phénoliques, de la xanthone et leurs dérivés, des caroténoïdes et/ou des tri-terpènes.

5. Composition pharmaceutique selon l'une quelconque des revendications 2 à 4, comprenant également un ingrédient actif supplémentaire.

6. Ensemble, comprenant une ou plusieurs compositions pharmaceutiques, dont au moins une contient un extrait selon la revendication 1.

7. Procédé de fabrication d'un extrait selon la revendication 1, comprenant les étapes suivantes :
- l'extraction de matière de feuilles et d'écorce et/ou de racines de *Hornstedtia scyphifera* avec du méthanol ;
- la séparation de l'extrait de méthanol avec un mélange 1:1 de chloroforme et d'eau ;
- l'enlèvement de la matière non dissoute ;
- la libération de l'extrait de chloroforme par rapport au solvant et la séparation de l'extrait avec un mélange 1:1 d'hexane et de méthanol aqueux à 20 % ;
- l'enlèvement de la matière non dissoute ;
- la division du reste en un extrait d'hexane et un extrait de méthanol aqueux ;
- le séchage de l'extrait méthanolique et la décomposition de l'extrait sec dans de l'éthanol.

8. Extrait selon la revendication 1, destiné à être utilisé comme ingrédient actif d'un médicament.

9. Extrait selon la revendication 1, destiné à être utilisé dans le traitement ou la prophylaxie d'une maladie.

10. Extrait selon la revendication 1 en tant que supplément alimentaire.

11. Extrait selon la revendication 1 ou composition pharmaceutique selon l'une quelconque des revendications 2 à 5 pour une utilisation dans la prophylaxie ou le traitement d'une maladie inflammatoire et/ou neurologique ou en tant que supplément alimentaire.

12. Extrait selon la revendication 1 ou composition pharmaceutique selon l'une quelconque des revendications 2 à 5 pour une utilisation dans la prophylaxie ou le traitement d'une maladie auto-immune ou d'un rejet de greffe.

13. Composition pharmaceutique selon l'une quelconque des revendications 11 à 12 pour une utilisation en association avec un ou plusieurs agents thérapeutiques supplémentaires.
